# EUROPEAN PATENT APPLICATION

(11) **EP 4 292 602 A1**
(43) Date of publication of application: **20.12.2023**
(21) Application number: 22305867.8
(22) Date of filing: 14.06.2022
(51) Int. Cl.: A61K 38/17, A61K 9/00, A61K 39/395, A61P 31/00, A61P 31/12, A61P 31/14

(54) **INTRANASAL ADMINISTRATION OF A POLYCLONAL BLOOD DERIVATIVE DIRECTED AGAINST A PATHOGEN AGENT FOR THE PREVENTION AND/OR TREATMENT OF AN AIRBORNE DISEASE**

(71) Applicant: PREVOR INTERNATIONAL, 75015 Paris (FR)
(72) Inventor: BLOMET, Joël, 95760 Valmondois (FR); DESMECHT, Daniel, 4141 Sprimont (BE)
(74) Representative: Plasseraud IP

(57) **Abstract**

The present invention relates to a blood derivative comprising polyclonal antibodies directed against a pathogen agent, for use in prevention and/or treatment of an airborne disease caused by said pathogen agent, characterized in that the blood derivative is administered intranasally.

## Description

The present invention relates to the field of serotherapy or seroprophylaxis, more particularly to the use of a serum or a plasma comprising polyclonal antibodies for the prevention and/or the treatment of an airborne disease, such as Covid-19. Said serum is administered intranasally.

Diseases can be transmitted by direct contact, inhalation, swallowing, biting or through the venereal route. The most communicable diseases are generally transmitted through the respiratory route, such as airborne disease which are often highly contagious.

A given pathogen can be characterized by its virulence, transmissibility and/or ability to evade immunity acquired through previous infection or vaccination. The virulence of a pathogen is usually linked to the severity of the disease, hence linked to morbidity and mortality.

Protecting against the severity of an infectious disease can be done preventively or curatively, as illustrated with the Covid-19 pandemic which has given rise to unprecedented research, particularly to develop vaccines and treatments.

For example, the vaccines developed by Pfizer/BioNTech, Moderna, J&J and Astra-Zeneca protect very well against the severity of the disease associated with subsequent infection with the SARS-CoV-2. Also, monoclonal antibodies, Lagevrio^{®}/molnupiravir (Merck) and Paxlovid^{®}/ritonavir (Pfizer) administered curatively promise to reduce the severity of Covid-19.

The contagiousness of an infection can significantly affect society in its functioning because too many simultaneous infections exert unsustainable pressure on health systems. Hindering contagion requires distancing, wearing masks, limiting densities in closed spaces, or even a lock down, .... These measures nevertheless have an unsustainable economic impact in the long term. In addition, there are always places whose densities can hardly be reduced without blocking society, for example public transport, schools, ... Moreover, even if vaccines against several airborne diseases already exist, accessibility to the vaccines can be difficult (for example in developing countries).

In certain particular cases, contagion is hampered by the spontaneous or post-vaccination immune response called "sterilizing" immunization. However, this situation is very rare. For example, in the case of Covid-19, people cured of a previous infection with the SARS-CoV-2 can be infected again, as well as vaccinated people. Admittedly, they can remain a- or pauci-symptomatic but they replicate the virus, disseminate it in the environment and can transmit it to one or more third parties.

Every day, more than 650,000 new cases of Covid-19 are recorded worldwide. Although Covid-19 vaccines have been shown to be safe and effective, 90% of experts expect Covid-19 to become endemic¹. Thus, the SARS-CoV-2 virus will infect millions of people each year, which could lead to new epidemics due to its proven ability to mutate into more infectious or more transmissible variants.

On one hand, vaccines against Covid-19 are only partially effective² (60-90%), their effectiveness decreases over time³ and a significant portion of the population will not be vaccinated for medical or personal reasons. On the other hand, the drugs under development or authorization for the treatment of Covid-19 are very expensive⁴ and are therefore not suitable for the treatment of the moderate form of the disease.

Finally, and generally speaking, since primary infections and vaccines do not offer sterilizing immunization (or rarely) against an airborne disease, there is no solution other than distancing, hand hygiene and wearing a mask to prevent contagion. This is also true if no vaccines are available or if the number of people vaccinated is too low (such as for example in developing countries).

To date, there are no safe and cost-effective drugs for the prevention/treatment of an airborne disease, such as Covid-19, but also influenza, common cold, measles... A safe and cost-effective solution to effectively prevent/treat individuals before disease onset or in the event of moderate disease (such as moderate Covid-19) is also crucial for preventing new endemic infections or possible pandemics. A safe and cost-effective solution to effectively reduce or stop virus transmission, such as SARS-CoV-2 virus, is also needed.

There is therefore a need for new solutions to prevent and/or treat even more efficiently airborne diseases, such as Covid-19. There is also an urgent need for a safe solution that can be used as self-medication, non-invasive, to relieve symptoms and, above all, to prevent contagion and/or reduce or stop virus transmission. Ideally this new solution should be easily implemented industrially and cheap to be accessible to as many people as possible. Such a solution should also be able to maintain its anti-contagion activity against the possible variants of the pathogen agent responsible to the airborne disease, such as the different SARS-CoV-2, influenza virus type A, ...

The present invention is based on the Inventors results proving that these needs are fulfilled by intranasally administering polyclonal antibodies, such as anti-SARS-CoV-2 polyclonal antibodies.

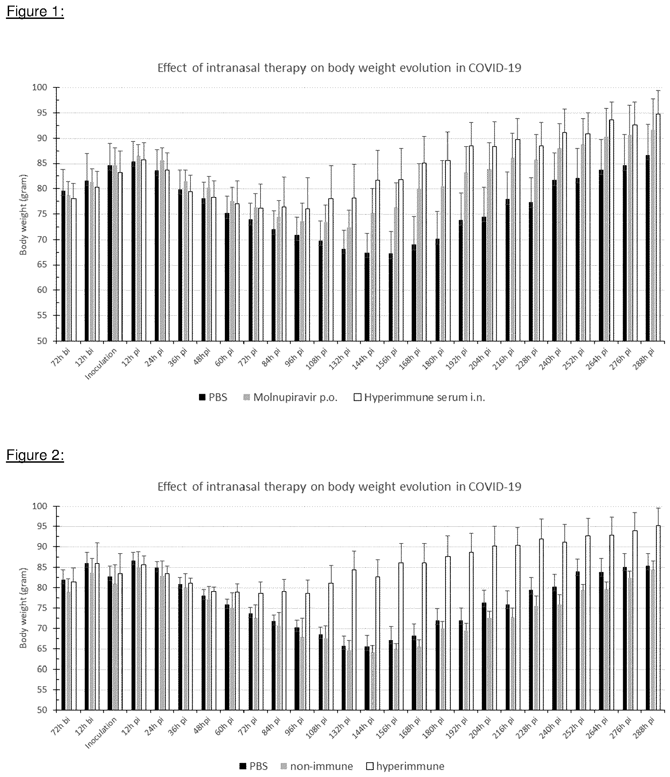

The administration of anti-SARS-CoV-2 polyclonal antibodies by intranasal route has already been described by Kangro *et al.*⁵*.* However, Kangro *et al.* have administered a bovine colostrum derived polyclonal antibodies against SARS-CoV-2 whereas in the present invention this is a blood derivative, such as a serum, which is administered. The administration of a blood derivative, such as a serum, as in the invention, provides substantial benefits. Indeed, an intranasal administration of a polyclonal blood derivative, such as serum, against SARS-CoV-2 can prevent and/or treat Covid-19, whereas in Kangro *et al.* the formulation derived from colostrum is only intended for prophylactic purpose. Moreover, a blood derivative, such as a serum, is easy to obtain (even more compared to getting colostrum), easy to administer, providing an easy and cost-effective solution for everyone. Furthermore, the results prove that the blood derivative (serum) of the invention is also much more efficient by comparison to the bovine colostrum derived polyclonal antibodies against SARS-CoV-2. Indeed, in Kangro *et al.* the colostrum has a neutralizing titer of about 25 (see Figure 2C of Kangro *et al.*) whereas in the present invention the neutralizing titer is at least about 160 when the blood derivative (serum) has been collected from rabbit and at least about 640 when the blood derivative (serum) has been collected from hamster (see Examples 1-3). Moreover, in Kangro *et al.* the bovine IgG concentration is decreased from 5.65 µg/mL to 2.36 µg/mL from 1 to 4 hours after administration, which means that the application of the nasal spray containing bovine colostrum derived polyclonal antibodies must be applied every 3-4 hours, whereas Example 1 of the invention proves that one-daily administration is sufficient.

Agurto-Arteaga *et al.*⁶ also discuss preclinical assessment of IgY antibodies against recombinant SARS-CoV-2 RBD protein for prophylaxis and post-infection treatment of COVID-19. Agurto-Arteaga *et al.* have administered egg-yolk antibodies against a recombinant protein of SARS-CoV-2 whereas in the present invention this is a blood derivative, such as a serum, which is administered. Moreover, and contrary to Agurto-Arteaga *et al.,* the present invention proves that an intranasal administration of a polyclonal blood derivative, such as serum, against SARS-CoV-2 can also reduce or stop the transmission of the virus (see Examples 5-6).

Therefore, the present invention relates to a blood derivative comprising polyclonal antibodies directed against a pathogen agent, for use in prevention and/or treatment of an airborne disease caused by said pathogen agent, characterized in that the blood derivative is administered intranasally.

The term "blood derivative" refers to any fraction issued from whole blood which can comprise polyclonal antibodies directed against a pathogen agent.

According to an embodiment of the invention, the term "blood derivative" as used herein refers to serum or plasma, preferably to serum.

Therefore, in a preferred embodiment, the present invention relates to a serum comprising polyclonal antibodies directed against a pathogen agent, for use in prevention and/or treatment of an airborne disease caused by said pathogen agent, characterized in that the serum is administered intranasally.

The term "serum" as used herein refers to the liquid fraction of whole blood, after a clot, which is usually collected after centrifugation. Therefore, serum usually corresponds to the supernatant obtained after centrifugation. The term "plasma" as used herein refers to the liquid fraction of whole blood collected in presence of an anticoagulant. In other words, serum refers to blood serum and plasma refers to blood plasma. According to an embodiment of the invention, serum does not contain clotting factors, fibrinogen, white blood cells, red blood cells or platelets. According to an embodiment of the invention, plasma does not contain white blood cells, red blood cells or platelets. Preferably, said blood derivative, serum or plasma contains polyclonal antibodies, electrolytes, hormones, ... According to the invention, the blood derivative, such as serum, does not comprise the serum complement or comprise an inactivated serum complement. For example, the complement can be inactivated by heat and a complement-depleted serum can be prepared by immunoadsorption, membrane filtration, ... According to an embodiment of the invention, blood derivative such as serum can also contain at least one antigen. In such a case, a step of separation can be carried out to remove the said antigen from the blood derivative. Preferably the blood derivative is hyperimmune. Preferably, the serum refers to a hyperimmune serum. As used herein, the term "hyperimmune refers to a high concentration of antibodies produced in reaction to repeated exposures to, contacts with or injections of an antigen (of a pathogen agent or part thereof). Consequently, a "hyperimmune serum" refers to a serum containing a high concentration of antibodies produced in reaction to repeated exposures to, contacts with or injections of an antigen (of a pathogen agent or part thereof). As used herein, a "high concentration of antibodies" refers to a neutralizing titer 50 of at least 80, at least 160, preferably at least 320; for example, a "serum containing a high concentration of antibodies" refers to a serum which has a neutralizing titer 50 or NT50 of at least 80, at least 160, preferably at least 320.

As used herein, the term "pathogen agent" refers to any infectious agent capable of causing an airborne disease in its host. In other words, said pathogen agent is responsible for the airborne disease. The term "pathogen agent" encompasses any variants of said pathogen variant. For example, it can be a virus, bacteria, fungi or yeast. Without being limitative, such pathogen agent encompasses coronavirus (such as SARS-CoV virus, SARS-CoV-2 virus, MERS-CoV virus), influenza virus (such as influenza A virus subtype H1N1 or H3N2), measles virus, adenovirus, mumps virus, rotavirus, poxvirus, enterovirus, rhinovirus, hantavirus, varicella zoster virus, *Mycobacterium tuberculosis, Bacillus anthracis, Bordetella pertussis, Neisseria meningitidis, Streptococcus pneumoniae,* fungi of the genus *Aspergillus* (such as *Aspergillus fumigatus, Aspergillus flavus, Aspergillus nidulans, Aspergillus versicolor, Aspergillus niger* and *Aspergillus terreus),* bacteria belonging to *Legionellaceae* family (such as *Legionella pneumophila*), *Blastomyces dermatitidis* yeast of the genus *Cryptococcus* (such as *Cryptococcus neoformans*), ...

The term "airborne disease" as used herein relates to a disease that is caused by a pathogen agent that is transmitted through the air, notably through small respiratory droplets. Such pathogen agent can be of any type and can be transmitted through inhalation, sneezing, coughing, talking or any other action that can generate aerosolized particles and/or contaminate surfaces. Usually, the aerosolized particles originate from the body secretions of an infected subject, but they can also originate from an animal. Preferably, the airborne disease is chosen among *Bacillus anthracis* infection, aspergillosis, blastomycosis, chickenpox, adenovirus infection, enteroviruses infection, rhinovirus infection, rotavirus infection, influenza, meningitis, legionellosis, measles, mumps, smallpox, cryptococcosis, tuberculosis, pertussis, streptococcus pneumoniae infection, severe acute respiratory syndrome, middle east respiratory syndrome or Covid-19. Preferably, such disease is Covid-19.

Therefore, in an embodiment, the present invention relates to a blood derivative comprising polyclonal antibodies directed against SARS-CoV-2, for use in prevention and/or treatment of Covid-19, characterized in that the blood derivative is administered intranasally.

In a preferred embodiment, the present invention relates to a serum comprising polyclonal antibodies directed against SARS-CoV-2, for use in prevention and/or treatment of Covid-19, characterized in that the serum is administered intranasally.

As used herein, the term "polyclonal antibodies directed against a pathogen agent" means that the antibodies can bind to said pathogen agent or part thereof.

As used herein, "polyclonal antibodies" refers to a mixture of different antibodies and/or fragments thereof which bind to different immunogenic determinant within the antigen (e.g. antigen of the pathogen agent, such as SARS-CoV-2 antigen). As used herein, the term "fragments" refers to any molecule or part of an antibody which is capable to bind to the antigen or part thereof. For example, such fragments are Fab, scFv, (scFv)₂, F(ab')₂, VHH or nanobodies. The antibodies within the blood derivative, such as the serum, of the invention bind to different epitopes of said antigen. This is advantageous because it confers a cross-protection and therefore the blood derivative, such as the serum, according to the invention is not sensitive to genetic evolution of the pathogen agent, such as a virus, preferably SARS-CoV-2. In other words, the blood derivative, such as the serum, of the invention maintains its efficiency, even when and if the pathogen agent, such as a virus, preferably SARS-CoV-2 virus, continues to mutate. This is a crucial advantage by comparison with monoclonal antibodies. Preferably, the polyclonal antibody specifically binds to an antigen of the pathogen agent. Preferably, the polyclonal antibody specifically binds to SARS-CoV-2 antigen.

As used herein, the term "prevention" refers to the prophylaxis of the airborne disease, preferably Covid-19. The blood derivative, such as the serum, according to the invention prevents or limits the risk of developing the airborne disease, preferably the Covid-19. The blood derivative, preferably the serum, according to the invention also prevents or limits or stops the contagion or transmission of the pathogen agent, such as SARS-CoV-2. In a prefer embodiment, the blood derivative, such as the serum, according to the invention prevents or limits or stops the contagion or transmission of the SARS-CoV-2. Therefore, the present invention if perfectly suitable before risk exposure.

As used herein, the term "treatment" refers to the therapy of the airborne disease, preferably Covid-19. For example, when a person is contaminated/infected by the pathogen agent, for example a virus such as SARS-CoV-2, the blood derivative, such as serum, according to the invention can be used for the alleviation of symptoms and/or to prevent for the evolution of a moderate disease to severe forms of the disease.

Preferably, the blood derivative, preferably the serum, of the invention prevents from docking of the pathogen agent to its cell surface receptor. In an embodiment of the invention, the blood derivative, such as the serum, of the invention prevents from docking of SARS-CoV-2 virus to its cell surface receptor, such as ACE2, NRP1, AXL, TIM1 and/or TIM4, preferably ACE2. Therefore, polyclonal antibodies act on the surface of upper airways, such as the lining epithelium of the nasal mucosa: in no case do they penetrate the cells.

The term "SARS-CoV-2" as used herein means the virus belonging to the Coronaviridae family, to the genus Betacoronavirus, and to the subgenus Sarbecovirus. It is an enveloped virus with a helical capsid whose genome consists of single-stranded RNA of approximately 30,000 nucleotides. The structure of the SARS-CoV-2 virion is notably as follows: it contains a helical capsid formed of protein N, a matrix formed of protein M and a lipid envelope in which at least two types of protein are embedded: the (glyco)protein S (spike) and small envelope protein (E). More particularly, the term "SARS-CoV-2" according to the invention refers to the virus whose sequence was initially described in GenBank, under the accession code MN908947, as well as all the variants of this virus. Thus, the term "SARS-CoV-2" has to be understood as meaning "SARS-CoV-2 virus and its variants". For example, the variants refer to the so-called Alpha (B.1.1.7), Beta (B.1.351), Gamma (P.1), Delta (B.1.617.2) and Omicron (B.1.1.529) variants, as well as the variants B.1.640, B.1.427, B.1.429, B.1.616, B.1.525, P.3, B.1.617.2, B.1.620, B.1.617.3, B.1.214.2, A.23.1, A.27, A.28, C.16, B.1.351, B.1.526, B.1.526.1, B.1.526.2, P.2, B.1.1.519, AV.1, AT.1, C.36, B.1.621, C.37, AY.4.2, B.1.1.318, B.1.617.2, C.1.2, BA.2. More information about SARS-CoV-2 sequence, mutations, variants can be found on https://www.ncbi.nlm.nih.gov/sars-cov-2/ ; https://www.cdc.gov/coronavirus/2019-ncov/cases-updates/variant-surveillance/variant-info.html ; https://www.gisaid.org/ or https://www.ecdc.europa.eu/en/covid-19/variants-concern.

As used herein, the term "Covid-19" refers to the disease caused by SARS-CoV-2 virus (Severe Acute Respiratory Syndrome CoronaVirus 2) and its variants.

As used herein, the term "intranasal" refers to delivery of the blood derivative, preferably the serum, to any portion of the upper airways, such as nose, pharynx, larynx and bronchi. Preferably, it refers to nasal mucosa, more preferably any portion of the nasal epithelium.

All antibodies have a common core structure of two light chains and two heavy chains. One light chain is attached to each heavy chain, and the two heavy chains are attached to each other to form a "Y" shaped molecule. However, despite this structural similarity the antibodies (also called immunoglobulins) can be divided into several isotypes and subtypes according to physiological, physiochemical properties. In mammals, the isotypes of antibody are named IgA, IgG, IgD, IgE and IgM. IgG isotype can also be divided into IgG1, IgG2, IgG3 and IgG4 and IgA isotype can be divided into IgA1 and IgA2. Camelid heavy chains only antibodies (HCAbs) are also encompassed in the present invention.

In an embodiment of the invention, said blood derivative, preferably the serum, comprises at least polyclonal antibodies of type IgA and IgG, and in a preferred embodiment said blood derivative, preferably the serum, comprises polyclonal antibodies of type IgA, IgM and IgG. In an embodiment of the invention, said blood derivative, preferably the serum, can also comprise polyclonal antibodies of type of IgA, IgG, IgM, IgD and IgE. Preferably said blood derivative, preferably the serum, comprises polyclonal antibodies of isotype IgG1, IgG2, IgG3 and IgG4 and/or of isotype IgG1, IgG2, IgG, IgG4, IgA1 and IgA2.

In an embodiment of the invention, the antibodies within the blood derivative, preferably the serum, are neutralizing antibodies. Such neutralizing antibodies act on the surface of upper airways, such as nasal mucosa, and neutralizes the pathogen agent, such as SARS-CoV-2, which cannot bind anymore to its receptor (such as ACE2) and penetrate the cells. Such antibodies remain in the upper airways, for example on the epithelium of nasal mucosa.

An antibody can be divided into two fragments: the region binding to the antigen (Fab region) and the tail region which activates the immune system (Fc region). Fc region of an antibody possess amino acids on which N-glycans (oligosacharides) can be linked. According to the invention, the Fc region of the polyclonal antibodies can be linked to at least one oligosaccharide. In a preferred embodiment, such oligosacharride is not only N-GlycolylNeurAminic acid (NGNA). It can be N-AcetylNeurAminic acid (NANA) or a mixture of N-GlycolylNeuraminic acid and N-AcetylNeurAminic acid. Therefore, in an embodiment, the present invention relates to a blood derivative, preferably a serum, comprising polyclonal antibodies directed against a pathogen agent, for use in prevention and/or treatment of an airborne disease, characterized in that the blood derivative, preferably the serum, is administered intranasally and in that the antibodies have a Fc region which is linked to (i) NANA or to (ii) NANA and NGNA. In a preferred embodiment, the present invention relates to a blood derivative, preferably a serum, comprising polyclonal antibodies directed against SARS-CoV-2, for use in prevention and/or treatment of Covid-19, characterized in that the blood derivative, preferably the serum, is administered intranasally and in that the antibodies have a Fc region which is linked to (i) NANA or to (ii) NANA and NGNA.

The blood derivative, preferably the serum, of the invention can be obtained from any mammals, preferably except bovine, sheep, goat. In a preferred embodiment of the invention, said blood derivative, preferably the serum, is from human, rabbit, chicken, pig, camelid and/or hamster, preferably rabbit and/or chicken.

Blood derivative, preferably the serum, can be obtained after infection of a subject with a pathogen agent, such as SARS-CoV-2. For example, blood derivative, preferably the serum, can be collected 10 and 100 days post-infection, preferably between 14 and 40 days. The expression "infection of a subject" means that the subject has been naturally contaminated/infected by a pathogen agent, such as SARS-CoV-2, or at least one of its antigens, and has developed polyclonal antibodies against the pathogen agent, such as SARS-CoV-2, but it also means that the subject has been contaminated/infected on purpose with the pathogen agent, such as SARS-CoV-2, or at least one of its antigens, in order to develop polyclonal antibodies against the pathogen agent, such as SARS-CoV-2. The contamination/infection step can be considered as an immunization step. The contamination/infection can be performed by any means, such as inhalation, intravenous injection, ... Blood derivative, preferably the serum, can also be obtained by immunization (or hyperimmunization) or vaccination of a subject against the pathogen agent, for example by injecting an attenuated virus, or a protein of the virus, or an RNA coding a protein of the virus, ... so that the subject develops polyclonal antibodies against the pathogen agent (or part thereof).

In an embodiment of the invention, said blood derivative, preferably the serum, has been obtained after infection of a subject with a pathogen agent which is identical or different to the variant of the same pathogen agent which is responsible or susceptible to be responsible to the airborne disease in the subject to be treated or prevented. Preferably, in an embodiment of the invention, said blood derivative, preferably the serum, has been obtained after infection of a subject with a SARS-CoV-2 which is identical or different to the SARS-CoV-2 which is responsible or susceptible to be responsible to Covid-19 in the subject to be treated or prevented. Indeed, a blood derivative, preferably the serum, collected after contamination/infection by one variant of SARS-CoV-2 does not limit its use for the prevention and/or treatment against the same variant. Due to the polyclonal nature of the antibodies within the blood derivative, preferably the serum, a blood derivative, preferably the serum, collected after contamination/infection by one variant of SARS-CoV-2 (for example B.1.1.7) can be used for the prevention and/or treatment against the other types of variants (for example B.1.1.529).

Moreover, the mammal in which the blood derivative, preferably the serum, is prepared does not limit its use for the same mammal. Indeed, a blood derivative, such as serum, collected after contamination/infection of one mammal (for example a hamster) can be used for the prevention and/or treatment of another mammal (for example a human): this is a heterologous polyclonal serotherapy. A blood derivative, such as serum, collected after contamination/infection of one mammal (for example a hamster) can also be used for the prevention and/or treatment of the same mammal (here a human): this is a homologous polyclonal serotherapy.

In an embodiment of the invention, said blood derivative is a purified blood derivative, preferably a purified serum.

In an embodiment of the invention, said blood derivative, such as serum, further comprises a pharmaceutical acceptable vehicle. Said blood derivative, such as serum, can also comprises additional substances, such as one or more excipients or another substance, for example to adjust pH, to increase half-life of said antibodies in nasal mucus, to modify the solubility of said antibodies, ...

In a preferred embodiment of the invention, said blood derivative, such as serum, is administered at least once-daily. Said blood derivative, such as serum, can also be administered twice-daily. Such blood derivative, such as serum, can be administered once every 24 hours or twice every 12 hours. In an embodiment of the invention, the blood derivative, such as serum, of the invention is administered in both nostrils.

In an embodiment of the invention, said blood derivative, such as serum, is administered on the nasal mucosa.

In an embodiment of the invention, said blood derivative, such as serum, is liquid, lyophilized or in any powder form.

In an embodiment of the invention, said blood derivative, such as serum, is administered by spray, powder, drop, aerosol, or nebulization.

In a preferred embodiment of the invention, said blood derivative, such as serum, is comprised within a nasal spray or a powder.

The blood derivative, such as serum, of the invention comprises polyclonal antibodies at therapeutic effective quantities. In an embodiment of the invention, said blood derivative, such as serum, has a neutralizing titer 50 of at least 80, preferably 320. As used herein, "neutralizing titer 50" or NT50 refers to the highest dilution of serum that prevents 50% of the infection *in vitro.*

In an embodiment of the invention, said blood derivative, such as serum, is administered to a subject, preferably a human or an animal. Said subject can be vaccinated against the airborne disease, such as Covid-19, or not vaccinated against the airborne disease, such as Covid-19. For example, a subject "vaccinated against an airborne disease, such as Covid-19" means that the subject has received at least one injection of a vaccine against the airborne disease such Covid-19, at least two injections of a vaccine against the airborne disease such Covid-19 or at least three injections of a vaccine against the airborne disease such Covid-19.

In an embodiment of the invention, the blood derivative, such as serum, as herein defined is comprised within a composition. Therefore, the present invention also relates to a composition comprising a blood derivative as herein defined, for use in prevention and/or treatment of an airborne disease caused by said pathogen agent, characterized in that said composition is administered intranasally.

In an embodiment, the present invention also relates to a method for preventing and/or treating of an airborne disease caused by a pathogen agent, comprising the intranasal administration of a therapeutically efficient amount of a blood derivative, such as serum, comprising polyclonal antibodies directed against said pathogen agent.

In an embodiment, the present invention also relates to a method for preventing and/or treating Covid-19 comprising the intranasal administration of a therapeutically efficient amount of a blood derivative, such as serum, comprising polyclonal antibodies directed against SARS-CoV-2. The present invention also relates to a method for preventing or reducing or stopping a virus transmission comprising the intranasal administration of a therapeutically efficient amount of a blood derivative, such as serum, comprising polyclonal antibodies directed against SARS-CoV-2.

In an embodiment, the present invention also relates to the use of blood derivative, such as serum, comprising polyclonal antibodies directed against a pathogen agent for the manufacture of a intranasal medicament for the prevention/treatment of an airborne disease, preferably Covid-19, caused by said pathogen agent. In an embodiment, the present invention also relates to the use of blood derivative, such as serum, comprising polyclonal antibodies directed against SARS-CoV-2 for the manufacture of an intranasal medicament for the prevention or reduction or stopping of SARS-CoV-2 transmission.

### References:

¹Torjesen, I. Covid-19 Will Become Endemic but with Decreased Potency over Time, Scientists Believe. BMJ 2021, 372, n494. https://doi.org/10.1136/bmj.n494
²Efficacy and protection, RIVM https://www.rivm.nl/en/covid-19-vaccination/vaccines/efficacy-and-protection (accessed 2021 -11 -02)
³Tartof, S. Y.; Slezak, J. M.; Fischer, H.; Hong, V.; Ackerson, B. K.; Ranasinghe, O. N.; Frankland, T. B.; Ogun, O. A.; Zamparo, J. M.; Gray, S.; Valluri, S. R.; Pan, K.; Angulo, F. J.; Jodar, L.; McLaughlin, J. M. Effectiveness of MRNA BNT162b2 COVID-19 Vaccine up to 6 Months in a Large Integrated Health System in the USA: A Retrospective Cohort Study. The Lancet 2021, 398 (10309), 1407-1416. https://doi.org/10.1016/S0140-6736(21)02183-8.
⁴Lupkin, S. Remdesivir Priced At More Than $3,100 For A Course Of Treatment. NPR. June 29, 2020.
⁵Bovine Colostrum Derived Antibodies Against SARS-CoV-2 Show Great Potential to Serve as a Prophylactic Agent. Kadri Kangro, Mihhail Kurašin, Kiira Gildemann, Eve Sankovski, Eva Zusinaite, Laura Sandra Lello, Raini Pert, Ants Kavak, Väino Poikalainen, Lembit Lepasalu, Marilin Kuusk, Robin Pau, Sander Piiskop, Siimu Rom, Ruth Oltjer, Kairi Tiirik, Karin Kogermann, Mario Plaas, Toomas Tiirats, Birgit Aasmäe, Mihkel Plaas, Dagni Krinka, Ene Talpsep, Meelis Kadaja, Joachim M. Gerhold, Anu Planken, Andres Tover, Andres Merits, Andres Männik, Mart Ustav Jr, Mart Ustav. doi: https://doi.org/10.1101/2021.06.08.21258069
⁶Preclinical assessment of IgY antibodies against recombinant SARS-CoV-2 RBD protein for prophylaxis and post-Infection treatment of COVID-19. Andres Agurto-Arteaga, Astrid Poma-Acevedo, Dora Rios-Matos, Ricardo Choque-Guevara, Ricardo Montesinos-Millan, Angela Montalvan, Gisela Isasi-Rivas, Yudith Cauna-Orocollo, Maria de Grecia Cauti-Mendoza, Norma Perez-Martinez, Kristel Gutierrez-Manchay, Ingrid Ramirez-Ortiz, Dennis Nuñez-Fernandez, Mario I. Salguedo-Bohorquez, Stefany Quiñones-Garcia, Manolo Fernandez Diaz, Luis A. Guevara Sarmiento, Mirko Zimic1,2 and COVID-19 Working Group in Peru. Front Immunol. 10 May 2022 May; Vol 13, Article 881604. doi: 10.3389/fimmu.2022.881604.

The invention will be illustrated by means of the following examples as well as the tables and figures, without being limitative.

### FIGURE LEGENDS

**Figure 1** represents the effect of intranasal therapy on body weight evolution in Covid-19 in hamsters having received once-daily administration of an hyperimmune serum obtained from a hamster.
**Figure 2** represents the effect of intranasal therapy on body weight evolution in Covid-19 in hamsters having received twice-daily administration of an hyperimmune serum obtained from a hamster.
**Figure 3** represents the effect of intranasal therapy on body weight evolution in Covid-19 in hamsters having received twice-daily administration of an hyperimmune serum obtained from a rabbit.
**Figure 4** represents the effect of intranasal therapy on body weight evolution in Covid-19 in hamsters having received twice-daily administration of an hyperimmune serum obtained from a human.

In the figures, "bi" means before inoculation and "pi" means post-inoculation.

### EXAMPLES

### Example 1: Once-daily intranasal homologous polyclonal serotherapy

Three groups of hamsters (n=10) were enrolled in the experiment.

One hundred microliters (100 µL) of a solution containing 10⁵ TCID₅₀/mL of SARS-CoV-2 (Alpha variant) virus was administered intranasally to the three groups. In addition, group 1 received 100 µL of PBS intranasally five times consecutively at 24-hour intervals beginning 24 hours after virus inoculation (black boxes). Group 2 received oral molnupiravir (an antiviral) once a day for 5 days, starting 24 hours after virus inoculation (grey boxes). Group 3 received 100 µL of a decomplemented hamster hyperimmune serum intranasally, again five times consecutively at 24-hour intervals beginning 24 hours after virus inoculation (white boxes). The hyperimmune serum pool was formed with sera collected from hamsters that had been infected with SARS-CoV-2 (Alpha variant) 14 days before bleeding (NT50=1/640). Animal weight is used here as an indicator of morbidity associated with experimental COVID-19. Once-daily intranasal serotherapy attenuates the magnitude, and shortens the duration, of weight loss associated with induced disease and outperforms molnupiravir in doing this.

The results are represented in **Figure 1** and show that once-daily intranasal homologous polyclonal serotherapy started 24 h after SARS-CoV-2 infection surpasses reference treatment molnupiravir in mitigating morbidity associated with COVID-19.

### Example 2: Twice-daily intranasal homologous polyclonal serotherapy

Three groups of hamsters (n=6) were enrolled in the experiment.

One hundred microliters (100 µL) of a solution containing 10⁵ TCID₅₀/mL of SARS-CoV-2 (Alpha variant) virus was administered intranasally to the three groups. In addition, group 1 received 100 µL of PBS intranasally ten times consecutively at 12-hour intervals starting 24 hours after virus inoculation (black boxes). Groups 2 and 3 received 100 µL of a decomplemented hamster non-immune (grey boxes) or hyperimmune (white boxes) serum intranasally using the same time scheme. The hyperimmune serum pool was formed with sera collected from 10 hamsters that had been infected with SARS-CoV-2 (Alpha variant) 14 days before bleeding (NT50=1/640).

Animal weight is used here as an indicator of morbidity associated with experimental COVID-19. Twice daily intranasal serotherapy suppresses the weight loss associated with induced disease.

The results are represented in **Figure 2** and show that twice-daily intranasal homologous polyclonal serotherapy started 24 h after SARS-CoV-2 infection suppresses morbidity associated with COVID-19.

### Example 3: Twice-daily intranasal heterologous polyclonal serotherapy

Three groups of hamsters (n=6) were enrolled in the experiment.

One hundred microliters (100 µL) of a solution containing 10⁵ TCID₅₀/mL of SARS-CoV-2 (Alpha variant) virus was administered intranasally to the three groups. In addition, group 1 received 100 µL of PBS intranasally ten times consecutively at 12-hour intervals beginning 24 hours after virus inoculation (black boxes). Groups 2 and 3 received 100 µL of decomplemented rabbit non-immune (grey boxes) or hyperimmune (black boxes) serum intranasally, respectively, using the same time scheme. The rabbit hyperimmune serum pool was formed by aggregating sera collected from 2 rabbits vaccinated with inactivated SARS-CoV-2 (Alpha variant) (NT50=1/160).

Animal weight is used here as an indicator of morbidity associated with experimental COVID-19. Twice-daily intranasal heterologous serotherapy suppresses disease-associated weight loss to an extent equivalent to the suppression conferred by homologous hyperimmune serum.

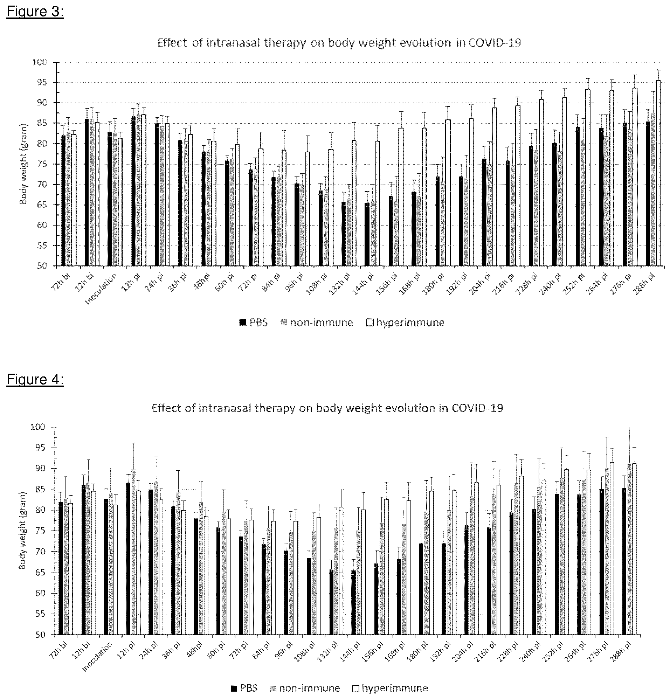

The results are represented in **Figure 3** and show that twice-daily intranasal heterologous polyclonal serotherapy started 24 h after SARS- CoV-2 infection suppresses morbidity associated with COVID-19.

Another hyperimmune serum pool from rabbits vaccinated with inactivated SARS-CoV-2 (Alpha variant) having neutralizing titer (NT50 on Vero E6 cells) against SARS-CoV-2 of 1/5120 and up to at least 1/40960 have also been obtained.

### Example 4: Twice-daily intranasal heterologous polyclonal serotherapy

Three groups of hamsters (n=6) were enrolled in the experiment.

One hundred microliters (100 µL) of a solution containing 10⁵ TCID₅₀/mL of SARS-CoV-2 (Alpha variant) virus was administered intranasally to the three groups. In addition, group 1 received 100 µL of PBS intranasally ten times consecutively at 12-hour intervals beginning 24 hours after virus inoculation (black boxes). Groups 2 and 3 received 100 µL of decomplemented human non-immune (grey boxes) or hyperimmune (black boxes) serum intranasally, respectively, using the same time scheme. The human hyperimmune serum pool was formed by aggregating sera collected from 2 human "vaccinated" with inactivated SARS-CoV-2 (Alpha variant) (NT50=1/160).

Animal weight is used here as an indicator of morbidity associated with experimental COVID-19. Twice-daily intranasal heterologous serotherapy suppresses disease-associated weight loss to an extent equivalent to the suppression conferred by homologous hyperimmune serum.

The results are represented in **Figure 4** and show that twice-daily intranasal heterologous polyclonal serotherapy started 24 h after SARS- CoV-2 infection suppresses morbidity associated with COVID-19.

### Example 5: A single homologous polyclonal serotherapy administered reduces the rate of transmission of SARS-CoV-2.

Three groups of hamsters (n=10) were enrolled in the experiment.

One hundred microliters (100 µL) of a solution containing 10⁵ TCID₅₀/mL of SARS-CoV-2 virus (Alpha variant) was administered intranasally to the first group (intentionally infected hamsters, referred to as "donors" below). One hundred microliters of either nonimmune or hyperimmune hamster serum was administered intranasally once to the other two groups ("recipient" hamsters) five minutes before their intimate exposure to the donors. Twenty-four or 28 hours after virus inoculation to the donors, each recipient was introduced into the cage of a donor for 4 hours. Then, the recipients were re-housed alone for 14 days. Seroconversion of animals to the SARS-CoV-2 nucleoprotein is used as an indicator of past infection. The seroconversion rate of each group reflects the success rate of between-hamsters transmission. H1, refers to hamster#1, H2, refers to hamster#2, etc. White boxes contain hamsters that had not seroconverted on day 14 post-infection (pi). Grey boxes refer to those that seroconverted (H11, H12, H8, H9, H10). Each donor was co-housed with 1 hamster of each treatment group (hyperimmune vs non-immune nasal spray). Co-housing was made 1:1 for 4 hours, either 24-28h after inoculation to donor or 28-32h after. Preventive intranasal serotherapy drastically decreases this seroconversion rate, and thus the inter-hamster transmission rate.

The results are represented in Table 1 below and show that a single homologous polyclonal serotherapy administered intranasally five minutes before co-housing with a contagious animal drastically reduces the rate of transmission of SARS-CoV-2.

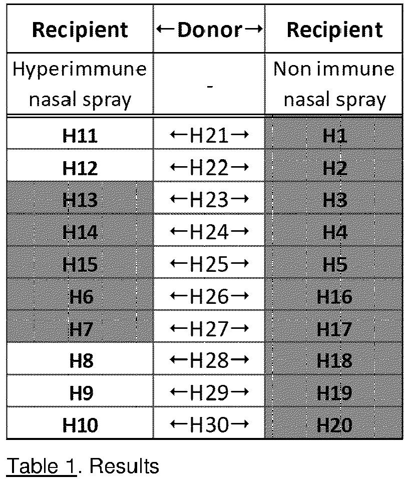

### Example 6: A single homologous polyclonal serotherapy administered intranasally reduces the morbidity associated with infection, if transmitted

Three groups of hamsters (n=10) were enrolled in the experiment.

One hundred microliters (100 µL) of a solution containing 10⁵ TCID₅₀/mL of SARS-CoV-2 virus (Alpha variant) was administered intranasally to the first group (intentionally infected hamsters, referred to as "donors" below). One hundred microliters of either nonimmune or hyperimmune hamster serum was administered intranasally once to the other two groups ("recipient" hamsters) five minutes before their intimate exposure to the donors. Twenty-four or 28 hours after virus inoculation to the first group, each recipient was introduced into the cage of a donor for 4 hours. Then, the recipients were re-housed alone for 14 days. The evolution of the weight of the animals (expressed as a percentage of the pre-contact body weight) over the 4 days after co-housing with a donor is used as an indicator of the severity of COVID-19 possibly contracted by inter-hamsters contagion. It is clearly seen that (1) COVID-19 is much less severe when contracted by inter-hamsters contagion than after intentional intranasal inoculation (donors showed an average weight loss of 16.2% over similar time window) and (2) a single intranasal serotherapy just before a risk taking event (co-housing with an infected mate here) reduces morbidity even if transmission of the virus takes place. Light grey boxes contain hamsters that had not seroconverted on day 14 post-infection (pi). Dark grey boxes refer to those that seroconverted. Each donor was co-housed with 1 hamster of each treatment group (hyperimmune vs non-immune nasal spray). Co-housing was made 1:1 for 4 hours, either 24-28h after inoculation to donor or 28-32h after. The results are represented in Table 2 below and show that a single homologous polyclonal serotherapy administered intranasally five minutes before co-housing with a contagious animal drastically reduces the morbidity associated with infection, if transmitted.

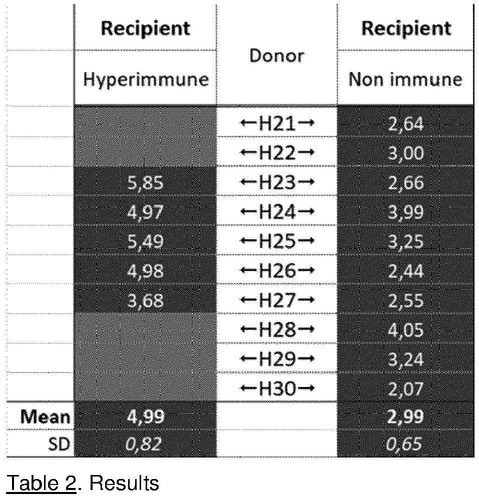

## Claims

1. Blood derivative comprising polyclonal antibodies directed against a pathogen agent, for use in prevention and/or treatment of an airborne disease caused by said pathogen agent, **characterized in that** the blood derivative is administered intranasally.

2. Blood derivative according to claim 1, wherein said blood derivative is serum or plasma, preferable serum.

3. Blood derivative according to any one of claims 1-2, wherein said blood derivative comprises at least polyclonal antibodies of type IgA and IgG, preferably IgA, IgM and IgG.

4. Blood derivative according to any one of claims 1-3, wherein said blood derivative comprises polyclonal antibodies of type IgA, IgG, IgM, IgD and IgE.

5. Blood derivative according to any one of claims 1-4, wherein said IgG are IgG1, IgG2, IgG3 and IgG4.

6. Blood derivative according to any one of claims 1-5, wherein said pathogen agent is SARS-CoV-2 and said airborne disease is Covid-19.

7. Blood derivative according to any one of claims 1-6, wherein said blood derivative is from human, chicken, rabbit, pig, camelid and/or hamster, preferably rabbit and/or chicken.

8. Blood derivative according to any one of claims 1-7, wherein said blood derivative has been obtained after infection, immunization and/or vaccination of a subject with a variant of said pathogen agent, such as SARS-CoV-2, which is identical or different to the variant of said pathogen agent, such as SARS-CoV-2, which is responsible or susceptible to be responsible to said airborne disease, such as Covid-19, in the subject to be prevented or treated.

9. Blood derivative according to any one of claims 1-8, wherein said blood derivative is administered by spray, powder, drop, aerosol or nebulization.

10. Blood derivative according to any one of claims 1-9, wherein said blood derivative is administered at least once-daily, preferably twice-daily.

11. Blood derivative according to any one of claims 1-10, wherein said blood derivative is comprised within a nasal spray or a powder.

12. Blood derivative according to any one of claims 1-11, wherein said blood derivative has a neutralizing titer 50 of at least 80, preferably 320.

13. Blood derivative according to any one of claims 1-12, wherein said blood derivative is administered to a subject, preferably a human or an animal.

14. Blood derivative according to any one of claims 1-13, wherein said subject is vaccinated against the airborne disease, such as Covid-19, or is not vaccinated against the airborne disease, such as Covid-19.

15. Composition comprising a blood derivative as defined in any one of claims 1-14, for use in prevention and/or treatment of an airborne disease caused by said pathogen agent, **characterized in that** said composition is administered intranasally.
